# EUROPEAN PATENT APPLICATION

(11) **EP 0 794 441 A2**
(43) Date of publication of application: **10.09.1997**
(21) Application number: 97301424.4
(22) Date of filing: 04.03.1997
(51) Int. Cl.: G02B 1/04, A61L 2/16

(54) **Contact lens containing a leachable absorbed antibody**

(30) Priority: 05.03.1996 US 611046
(71) Applicant: JOHNSON & JOHNSON VISION PRODUCTS, INC., Jacksonville, Florida 32216 (US)
(72) Inventor: Schultz, Clyde L., Ponte Vedra Beach, FL 32082 (US)
(74) Representative: Mercer, Christopher Paul

(57) **Abstract**

A contact lens made of a polymeric hydrogel material having absorbed therein as a leachable antibody, and a method for making the composition by soaking the polymeric material in a solution of the antibody.

## Description

### TECHNICAL FIELD

This invention relates to polymeric compositions employed to make contact lenses, the composition containing a polymeric hydrogel material and an antibody as a leachable material absorbed in the polymeric material.

### BACKGROUND OF THE INVENTION

It is known to employ hydrogel contact lenses to dispense therapeutic agents to the eye as disclosed in U.S. Patents Nos. 3,787,378; 4,668,506; 4,713,244; 4,484,922; 4,931,279; and 5,213,801. In European Patent Application No. 96309534.4 there is a disclosure of absorbing desferrioxamine into contact lenses as the medicinal agent to kill or inhibit the growth of bacteria. While some patents, e.g., U.S. Patent Nos. 3,957,049; 4,592,752; 4,668,506; and 4,713,244 have disclosed dispensing medicinal agents from contact lenses, none have considered dispensing an antibody from a contact lens to counterattack an antigen in the ocular liquids.

It is an object of this invention to provide a contact lens containing an absorbed antibody that is leachable into the liquid surrounding the eye. It is another object to provide a contact lens that is capable of leaching sufficient antibody, such as anti-Interleukin-1, into the ocular liquid to substantially inhibit the growth of an antigen, e.g., interleukin-1. Still other objects will become apparent from the more detailed description which follows.

### BRIEF DESCRIPTION OF THE DRAWINGS

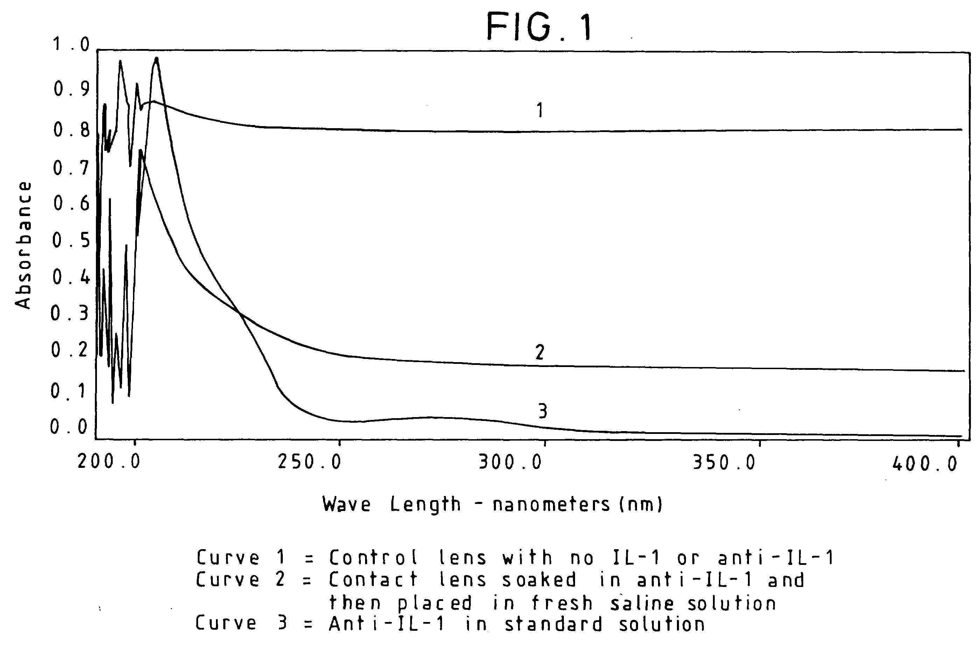

Figure 1 shows a chromatogram of released anti-IL-1, as described herein, from a contact lens.

### BRIEF SUMMARY OF THE INVENTION

This invention relates to a polymeric composition comprising a polymeric hydrogel material containing an antibody as an additive absorbed in the polymeric composition and leachable therefrom. The polymeric composition is especially useful as the material of a contact lens. A particularly advantageous embodiment of the invention is a contact lens of polymeric hydrogel material having an antibody, such as anti-interleukin-1, absorbed therein in an amount up to 400 mg/lens. The lenses which function best in this use are those that are hydrogels containing 52-60% water. At an ocular pH of about 7.0-7.4 about 40% of the absorbed antibody is releasable to the ocular liquid.

### DETAILED DESCRIPTION OF THE INVENTION

Antigens may be introduced into the body from a variety of sources outside the body. Typical antigens are toxins or enzymes. They might be introduced into the body through any orifice, such as the mouth, nose, eye, etc. Once the antigen is inside the body, the body naturally produces a counteracting substance to reduce or dispose of the antigen. Generally this naturally occurring counteraction results in the production of an antibody that opposes and/or neutralizes the antigen.

An antibody is a soluble product produced by stimulated immune cells. There are five classes of antibody. The most common antibody class used in medical research is IgG which has a molecular weight of 160,000 kd. The action of an antibody is specific in that only one type of antibody will bind to and deactivate or kill one particular bacteria or virus. An antibody which is produced against Pseudomonas aeruginosa will have no effect against Serratia marcescens even though both bacteria are Gram negative organisms. Furthermore, an antibody may be produced against any other immune product. This is done by injecting a purified protein such as IL-1 into a rabbit or some other animal. This animal's immune system will respond to the injected IL-1 as a foreign protein and develop an antibody response to the IL-1. The antibody which is specific for the IL-1 protein may be purified from the animal's serum, and thus one has an antibody capable of binding to and deactivating the IL-1 protein. As noted above, anti-IL-1 will have no direct effect on IL-6 or any other cytokine.

Once the IL-1 is stimulated, regardless of the stimulus, IL-1 will cause the side effects mentioned above.

A particularly important antibody for use in this invention is anti-IL-1 which is the antibody for IL-1. Interleukin-1 (IL-1 is a cytokine, or inflammatory mediator produced by living cells in response to a chemical stimulus such as a detergent, a pathogen, or a purified virulence factor such as lipopolysaccharide. Cytokines are proteins with variable molecular weights depending on the type of cytokine. Most molecular weights are between 17 and 20 kd. A virulence factor is a protein which may be purified and retain its pathogenic activity. Among the functions of cytokines is one which allows cells to communicate with one another. Different cytokines send different signals to different cells. This signaling system is turned on by a chemical stimulus and is cascading in effect. That is, a given cytokine, such as IL-1, will stimulate other cytokines, such as IL-2, IL-6, and IL-8. However, in this example the cascading effect is mutually exclusive to IL-1, that is IL-6 is not known to stimulate IL-8. For this reason, if one wished to interrupt this system, the optimal place would be at the early stage of the cascade. In this way, no other mediators would be stimulated. In addition, a cytokine may have an end process effect. It has been shown that topical inoculation of at least 100 pg in 50 microliters of IL-1 onto a rabbit cornea will cause ulceration within two hours. So, the action of cytokines is non-specific in nature (unlike a specific antibody/pathogen response) and a variety of different molecules may stimulate the cytokine response.

### EXAMPLE 1

*In vitro* experiments were conducted to determine if polymeric hydrogel contact lenses which had been impregnated with anti-Interleukin-1 could bind active interleukin-1 so that this molecule could be reduced in concentration and thus be unable to elicit a pathogenic response. Anti-interleukin-1 (anti-IL-1) is an antibody molecule which will selectively bind to IL-1 and prevent it from further function. The procedure of the experiment was as follows:
a. New contact lenses of acrylic polymer hydrogel were washed in physiological saline solution at pH 7.2.
b. The lenses were then exposed to 100 micrograms/mL of either rabbit or human anti-interleukin-1 (anti-IL-1) at 25 C for 24 hours.
c. The lenses were washed in saline solution at pH 7.2.
d. Antibody uptake and release were studied by incubation of lens materials with known solutions of anti-IL-1. After incubation, lenses were placed in saline solution for a specified period of time. Gel filtration chromatography was used to quantitate the anti-IL-1 content of the saline solution.
e. Sample lenses coded "experimental" or "control" were subjected *in vitro* analysis. The *in vitro* analysis was performed as follows. Human keratinocytes and fibroblasts which have the potential to produce IL-1 were stimulated chemically with 0.55 Triton X-100. Experimental lenses, coated with anti-IL-1 or uncoated control lenses were immediately placed into direct contact with keratinocytes and fibroblasts. Controls placed in the assay were lenses with no chemical stimulation to determine if the lens would cause IL-1 production by the cells. A positive control, with no lens and a negative control (no lens) were also added. In addition, an experimental lens was placed onto cells to determine if the lens/antibody combination would cause IL-1 release. Samples for assay were taken following two hours of incubation.
f. The IL-1 detection system used was an ELISA assay.

The results are shown in Table 1. These data show that lenses treated with anti-IL-1 significantly reduced the amount of detectable IL-1 from human keratinocytes and fibroblast cells as compared to untreated lenses (p = 0.002, one tailed t-value). Cells stimulated with 0.5% Triton X-100 produced detectable IL-1 at the same level as cells exposed to untreated control lenses with Triton X-100. When placed into culture alone, experimental lenses and control lenses did not stimulate production of detectable IL-1.

**Table 1**

| Run | Treatment | IL-1 detected pg/mL* |
|---|---|---|
| 1 | Experimental Lens + 0.5% Triton X-100 | 80.00 |
| 2 | Control Lens + 0.5% Triton X-100 | 420.00 |
| 3 | Experimental Lens + no Triton X-100 | 0.52 |
| 4 | Control Lens + no Triton X-100 | 14.00 |
| 5 | Positive Control + 0.5% Triton X-100 No Lens | 410.00 |

| | | |
|---|---|---|
| * pg/mL is picograms IL-1/milliliter cell culture fluid Run 1 is statistically significantly different from Runs 2 and 5 | | |

Figure 1 shows a chromatogram of released anti-IL-1 from a lens.

The results show a significant reduction in detected IL-1 when IL-1 producing cells are placed in direct contact with lenses treated with anti-IL-1. This means that should IL-1 be stimulated, an antibody released from the lens will prevent an end pathology such as a corneal ulcer. It has been shown that topical administration of IL-1 will cause corneal ulcers in rabbits. In addition, no toxicity was observed with either control lenses or experimental lenses (with antibody) in culture.

### EXAMPLE 2

An animal experiment was conducted to observe the effect of contact lenses releasing anti-IL-1 to prevent corneal ulceration induced from IL-1 in rabbits.

Fourteen animals were used in this study, split into two groups; a control group and an experimental group of seven animals each. All animals received corneal and limbal abrasions in one eye. The other eye was not used. Results were measured at one, two, and three hours post-application of the IL-1.

### Group 1. Control Group.

Following abrasion of the eye, 150 pg of rabbit IL-1 was applied to the eye. A standard hydrogel contact lens was then applied to the eye. Of the seven animals in this group which were exposed to IL-1 six of the animals (86%) developed corneal ulcers. All the animals (100%) developed chemosis, injection and severe tearing due to the IL-1 application.

### Group 2. Experimental Group.

Following abrasion of the eye, 150 pg of rabbit IL-1 was applied to the eye. A standard lens which had been treated with 100 micrograms/mL of rabbit anti-Interleukin-1, was applied to this eye. The results for this group showed zero of seven animals (0%) developed corneal ulcers. There was no chemosis or injection due to IL-1 application. There was slight tearing.

The results of this *in vivo* experiment show that an anti-inflammatory agent delivered from a hydrogel contact lens material can prevent corneal ulcers from developing. The concentration of IL-1 used to cause corneal ulcers to form was 50 micrograms higher than the minimum concentration required.

The foregoing examples relate to the antigen/antibody system of Interleukin-1/anti-Interleukin-1, but there are other antigen/antibody systems which are operable within this invention. For example, the amount of anti-IL-1 which is absorbed into a contact lens is about 100 mg. In general, this is accomplished by soaking the lens in IL-1 for about 24 hours, followed by washing the lens in saline solution at a pH of 7.2.

The release of anti-IL-1 from a lens previously treated with anti-IL-1 is at least about 150 pg.

While the invention has been described with respect to certain specific embodiments, it will be appreciated that many modifications and changes may be made by those skilled in the art without departing from the spirit of the invention. It is intended, therefore, by the appended claims to cover all such modifications and changes as fall within the true spirit and scope of the invention.

## Claims

1. A polymeric composition comprising a polymeric hydrogel material containing an antibody which is absorbed in the polymeric hydrogel material and capable of being leached out at ocular conditions.

2. The polymeric composition of Claim 1 wherein said antibody is anti-Interleukin-1.

3. The polymeric composition of Claim 1 or Claim 2 as a contact lens.

4. The polymeric composition of Claim 3 wherein the antibody is capable of being leached out to an extent of not more than about 400 mg antibody per lens.

5. A contact lens for an eye, the eye being in contact with ocular fluid, which contact lens is a polymeric composition containing an ionic acrylic polymeric material having absorbed therein an antibody, wherein the absorbed antibody is leachable into the ocular fluid at the existing conditions of the eye.

6. The contact lens of Claim 5 which contains anti-Interleukin-I in any amount up to about 100 mg/lens.

7. The contact lens of Claim 5 or Claim 6 wherein the polymeric composition is a polymeric hydrogel containing 35-60% water by weight.

8. The polymeric composition or contact lens of any one of Claims 1 to 7 wherein the polymeric material is a terpolymer of hydroxyethlmethacrylate, ethylene glycol dimethacrylate, and either acrylic acid or methacrylic acid.

9. The polymeric composition or contact lens of any one of Claims 1 to 8 wherein the ocular conditions include an ocular pH of 7.0-7.4.

10. A process for preparing a contact lens for use which comprises washing the lens in a saline solution; placing the washed lens in an aqueous solution of an antibody in a concentration of 100-250 mcg antibody/mL of solution at a pH of 7.0-7-4 for 60 to 80 hours; and removing the lens from the solution ready for use in the eye.
